(19) Europäisches Patentamt
European Patent Office
Office européen des brevets

(11) EP 1 214 309 B1

(12) **FASCICULE DE BREVET EUROPEEN**

(45) Date de publication et mention de la délivrance du brevet:
**10.03.2004 Bulletin 2004/11**

(21) Numéro de dépôt: **00960773.0**

(22) Date de dépôt: **01.09.2000**

(51) Int Cl.[7]: **C07D 311/66**, C07D 409/12

(86) Numéro de dépôt international:
**PCT/FR2000/002417**

(87) Numéro de publication internationale:
**WO 2001/017987 (15.03.2001 Gazette 2001/11)**

(54) **PREPARATION D'AMIDINES DERIVEES DE L'ACIDE 6-HYDROXY-2,5,7,8-TETRAMETHYLCHROMANE-2-CARBOXYLIQUE**

HERSTELLUNG VON AMIMIDE ABGELEITET VON 6-HYDROXY-2,5,7,8-TETRAMETHYLCHROMAN-2-SÄURE

PREPARING AMIDINES DERIVED FROM 6-HYDROXY-2,5,7,8-TETRAMETHYLCHROMANE-2-CARBOXYLIC ACID

(84) Etats contractants désignés:
**AT BE CH CY DE DK ES FI FR GB GR IE IT LI LU MC NL PT SE**

(30) Priorité: **03.09.1999 FR 9911044**

(43) Date de publication de la demande:
**19.06.2002 Bulletin 2002/25**

(73) Titulaire: **S.C.R.A.S.**
**75016 Paris (FR)**

(72) Inventeurs:
- **LE BRETON, Christine**
  **F-84000 Avignon (FR)**
- **MANGINOT, Eric**
  **F-84140 Montfavet (FR)**
- **CAZAUX, Jean-Bernard**
  **F-30390 Aramon (FR)**

(74) Mandataire: **Bourgouin, André**
**Beaufour Ipsen - S.C.R.A.S.,**
**24, rue Erlanger**
**75781 Paris Cedex 16 (FR)**

(56) Documents cités:
**FR-A- 2 761 066**
**FR-A- 2 764 889**
**FR-A- 2 783 519**

Remarques:
Le dossier contient des informations techniques présentées postérieurement au dépôt de la demande et ne figurant pas dans le présent fascicule.

EP 1 214 309 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un nouveau procédé pour la synthèse d'amidines dérivées de l'acide (-)-6-hydroxy-2,5,7,8-tétraméthylchromane-2-carboxylique.

Des arnidines dérivées de l'acide (-)-6-hydroxy-2,5,7,8-tétraméthylchromane-2-carboxylique, et en particulier le (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide, sont décrites dans les demandes de brevet PCT WO 98/42696 et WO 98/58934. Ces composés présentent l'avantage d'inhiber à la fois les NO Synthases (NOS) et la formation d'espèces réactives de l'oxygène (*Reactive Oxygen Species* ou ROS).

Un intermédiaire important dans la synthèse des amidines précitées est l'acide (-)-6-hydroxy-2,5,7,8-tétraméthylchromane-2-carboxylique. Des procédés de préparation de ce produit et de l'acide (-)-6-benzyloxy-2,5,7,8-tétraméthylchromane-2-carboxylique ont été décrits dans l'article suivant : Scott et al., *Journal of the American Oil Chemist's Society,* May 1974, 200-203. Ce produit est également commercialisé sous le nom de Trolox®.

La demanderesse a mis au point un nouveau procédé de préparation d'amidines dérivées de l'acide (-)-6-hydroxy-2,5,7,8-tétraméthylchromane-2-carboxylique, lequel utilise de nouveaux intermédiaires de synthèse. Ledit procédé présente l'avantage d'être utilisable à l'échelle industrielle et d'offrir aussi bien un très bon rendement global qu'un produit très pur.

La demanderesse a aussi développé un nouveau procédé de dédoublement de l'acide 6-benzyloxy-2,5,7,8-tétraméthylchromane-2-carboxylique, lequel permet d'obtenir facilement un excès énantiomérique supérieur à 99% pour l'un comme pour l'autre des énantiomères.

L'invention concerne tout d'abord les produits nouveaux de formule générale **(II)B**

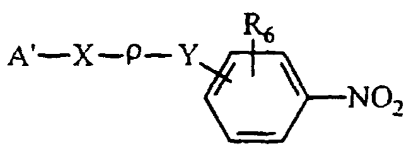

**(II)B**

sous forme racémique, d'énantiomères ou toute combinaison de ces formes,
formule générale **(II)B** dans laquelle

A' représente le radical

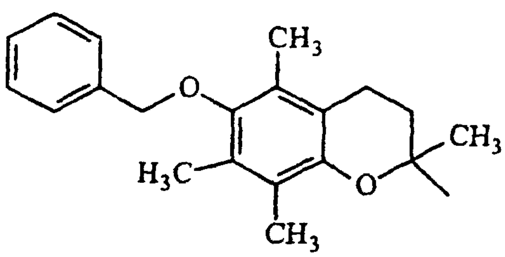

X représente $-Z_1-CO-$ ;

ρ représente une liaison ou un hétérocycle choisi parmi le groupe constitué des radicaux pipéridine, pipérazine, homopipérazine, 2-méthylpipérazine, 2,5-diméthyl-pipérazine et 4-aminopipéridine ;

Y représente un radical choisi parmi les radicaux $-Z_2-$ et $-NR_3-Z_2$,

$R_3$ représentant un atome d'hydrogène, un radical alkyle linéaire ou ramifié ayant de 1 à 6 atomes de carbone ou un radical $-COR_4$,

$R_4$ représentant un radical alkyle linéaire ou ramifié ayant de 1 à 6 atomes de carbone ;

$Z_1$ et $Z_2$ représentent indépendamment une simple liaison ou un radical alkylène linéaire ou ramifié ayant de 1 à 6 atomes de carbone, $Z_1$ et $Z_2$ représentant de préférence $-(CH_2)_m-$, m étant un entier compris entre 0 et 6 ;

$R_6$ représente un atome d'hydrogène ou un groupe OH.

Les produits de formule générale **(II)B** sont utiles à la préparation d'amidines de formule générale **(I)** décrite ci-après.

Les produits optiquement actifs peuvent être préparés par dédoublement de l'acide 6-benzyloxy-2,5,7,8-tétraméthylchromane-2-carboxylique, puis par des synthèses analogues à celles décrites dans les demandes de brevet PCT WO 98/42696 et WO 98/58934.

L'invention concerne donc également l'application des produits de formule générale (II)B précédemment décrits à la préparation d'amidines de formule générale **(I)**

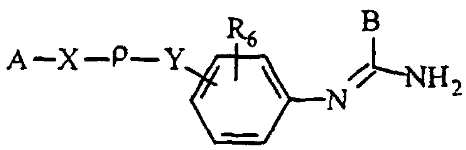

**(I)**

dans laquelle

A représente le radical

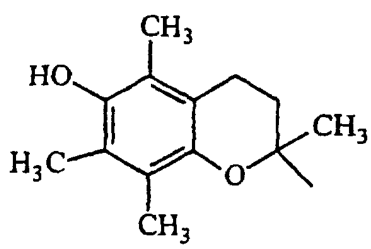

B représente un radical aryle carbocyclique ou aryle hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;

X représente $-Z_1$-CO- ;

ρ représente une liaison ou un hétérocycle choisi parmi le groupe constitué des radicaux pipéridine, pipérazine, homopipérazine, 2-méthylpipérazine, 2,5-diméthyl-pipérazine et 4-aminopipéridine ;

Y représente un radical choisi parmi les radicaux $-Z_2-$ et $-NR_3-Z_2$,

$R_3$ représentant un atome d'hydrogène, un radical alkyle linéaire ou ramifié ayant de 1 à 6 atomes de carbone ou un radical $-COR_4$,

$R_4$ représentant un radical alkyle linéaire ou ramifié ayant de 1 à 6 atomes de carbone ;

$Z_1$ et $Z_2$ représentent indépendamment une simple liaison ou un radical alkylène linéaire ou ramifié ayant de 1 à

6 atomes de carbone, $Z_1$ et $Z_2$ représentant de préférence $-(CH_2)_m-$, m étant un entier compris entre 0 et 6 ;

$R_6$ représente un atome d'hydrogène ou un groupe OH ;

application caractérisée en ce qu'elle consiste à faire subir au produit de formule générale **(II)B** les étapes successives suivantes :

1) réduction catalytique du composé nitro de formule générale **(II)B**

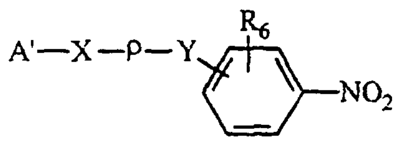

**(II)B**

dans laquelle

X, ρ, Y et $R_6$ ont la même signification que dans la formule générale **(I)** ;

et A'représente le radical

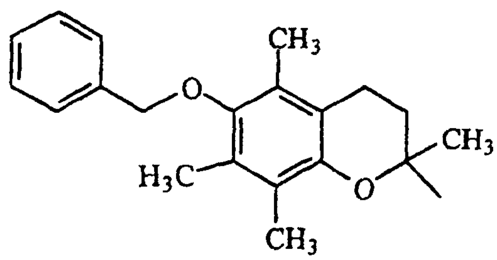

dans un alcool inférieur, de préférence tel que méthanol, éthanol ou alcool isopropylique, par action d'un donneur d'hydrogène en présence de Pd/C pour donner le composé amino de formule générale **(III)**

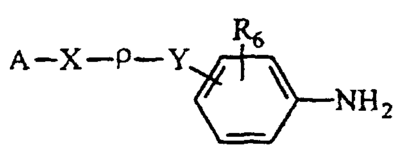

**(III)**

dans laquelle A, X, ρ, Y et $R_6$ ont la même signification que dans la formule générale **(I)** ;
2) réaction, dans un alcool inférieur, tel que méthanol, éthanol, alcool isopropylique ou t-butanol, de préférence dans l'alcool isopropylique, à une température comprise entre 20 et 90 °C, par exemple à 50 °C, et durant une à 48 heures, de préférence durant 15 à 24 heures, éventuellement en présence de DMF, du composé de formule générale **(III)** décrit précédemment

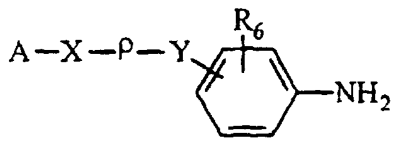

**(III)**

avec le composé de formule générale **(IV)**

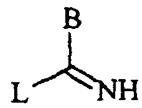

**(IV)**

dans laquelle B a la même signification que dans la formule générale **(I)** et L représente un groupe partant et notamment un radical alkoxy, alkylthio, aralkylthio, acide sulfonique, halogénure, alcool arylique ou tosyle (d'autres groupes partants bien connus de l'homme du métier et pouvant être éventuellement utilisés pour l'invention sont décrits dans l'ouvrage suivant : *Advanced Organic Chemistry*, J. March, 3rd Edition (1985), McGraw-Hill, p. 315), le composé de formule générale **(IV)** pouvant éventuellement être salifié par un acide minéral G. De préférence, G représente HCl, HBr ou HI.

Les composés de formule générale **(I)** contiennent un centre asymétrique et présentent des formes isomères. Le procédé selon l'invention permet d'obtenir le mélange racémique ou les énantiomères de ces composés suivant le choix d'un produit racémique ou optiquement actif comme produit de départ.

Les produits de formule générale **(I)** peuvent le cas échéant être obtenus sous forme de leurs sels et déplacés vers leur forme acide libre ou base libre, si besoin. Ils peuvent, au contraire, être obtenus sous leur forme acide libre ou base libre et être dans ce cas salifiés, si besoin. Des sels courants pour les produits de formule générale **(I)** sont notamment les hydrates, chlorhydrates, dichlorhydrates, fumarates et hémifumarates.

Les composés de l'invention peuvent exister à l'état de bases ou de sels d'addition notamment à des acides organiques ou inorganiques ou à des bases, et en particulier à l'état d'hydrates, de chlorhydrates, de dichlorhydrates, de fumarates ou d'hémifumarates.

Le composé de formule générale **(II)B** est obtenu selon des méthodes analogues à celles décrites dans les demandes de brevet PCT WO 98/42696 et WO 98/58934. La seule différence avec ces méthodes est que l'on utilise dans tous les cas le dérivé benzylé et non le dérivé hydroxylé de l'acide 6-hydroxy-2,5,7,8-tétraméthylchromane-2-carboxylique. L'acide 6-benzyloxy-2,5,7,8-tétraméthylchromane-2-carboxylique, sous forme racémique ainsi que sous ses deux formes énantiomères, peut être préparé par exemple selon les méthodes décrites ci-après dans les exemples.

Par ailleurs, l'homme du métier saura, le cas échéant, adapter les procédés ci-dessus en combinant les procédures décrites dans les demandes de brevet PCT WO 98/42696 et WO 98/58934 aux procédés ci-dessus ou à des étapes de ces procédés.

L'étape 1) du procédé décrit ci-dessus sera réalisée de préférence à une température supérieure à la température ambiante, et plus préférentiellement à une température proche du point d'ébullition du solvant alcoolique employé. Parmi les donneurs d'hydrogène utilisables, on pourra notamment citer l'acide formique, le formiate d'ammonium, le cyclohexadiène et le cyclohexène. De préférence, le donneur d'hydrogène sera le cyclohexène. En ce qui concerne le solvant, on pourra utiliser par exemple l'éthanol, le méthanol ou l'isopropanol. De préférence, la réaction sera réalisée dans l'éthanol.

De préférence, on emploiera comme produit de formule générale **(IV)** dans l'étape 2) du procédé défini ci-dessus le S-benzyl-(2-thiénylthioacétamidate) (pour sa préparation, cf. par exemple le brevet allemand DE 2358509). Plus préférentiellement, ledit S-benzyl-(2-thiénylthioacétamidate) sera utilisé sous une forme salifiée, de préférence à l'état de chlorhydrate (autrement dit, G représente de préférence HCl). Toujours dans l'étape 2) du procédé défini ci-dessus, le solvant préféré pour la réaction sera l'éthanol, la réaction se faisant de préférence à une température de 20 à 40 °C.

De préférence, le procédé ci-dessus sera utilisé pour préparer des produits de formule générale **(I)** pour lesquels B représente un radical thiophène.

On préférera tout particulièrement utiliser le procédé ci-dessus pour préparer le produit de formule générale **(I)** est le (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide.

L'invention offre par ailleurs un procédé de préparation de l'acide (-)-6-benzyloxy-2,5,7,8-tétraméthylchromane-2-carboxylique, caractérisé en ce qu'il comporte les étapes successives suivantes :

1) ajout, à une solution d'acide (±)-6-benzyloxy-2,5,7,8-tétraméthylchromane-2-carboxylique dans l'isopropanol, de L-(-)-phényléthylamine ;

2) chauffage à une température de préférence supérieure à 50 °C ;

3) refroidissement lent ;

4) récupération du solide formé par filtration ;

5) recristallisation dudit solide dans l'éthanol et récupération du produit recristallisé ;

6) traitement du produit obtenu par une solution aqueuse acide et récupération de l'acide (-)-6-benzyloxy-2,5,7,8-tétraméthylchromane-2-carboxylique après extraction de la phase aqueuse avec du *tert*-butylméthyléther.

L'invention offre de même un procédé de préparation de l'acide (+)-6-benzyloxy-2,5,7,8-tétraméthylchromane-2-carboxylique, caractérisé en ce qu'il comporte les étapes successives suivantes :

1) ajout, à une solution d'acide (±)-6-benzyloxy-2,5,7,8-tétraméthylchromane-2-carboxylique dans l'isopropanol, de L-(-)-phényléthylamine ;

2) chauffage à une température de préférence supérieure à 50 °C ;

3) refroidissement lent ;

4) élimination du solide formé par filtration ;

5) évaporation des solvants et récupération du solide obtenu ;

6) recristallisation dudit solide dans l'éthanol et récupération du produit recristallisé ;

7) traitement du produit obtenu par une solution aqueuse acide et récupération de l'acide (+)-6-benzyloxy-2,5,7,8-tétraméthylchromane-2-carboxylique après extraction de la phase aqueuse avec du *tert*-butylméthyléther.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporés par référence.

Les exemples suivants sont présentés pour illustrer les procédures ci-dessus et ne doivent en aucun cas être considérés comme une limite à la portée de l'invention.

## EXEMPLES

### Exemple 1 : acide (-)-6-benzyloxy-2,5,7,8-tétraméthylchromane-2-carboxylique

1.1) *(±)-6-benzyloxy-2,5,7,8-tétraméthylchromane-2-carboxylate de méthyle* :

A une suspension de 1,34 kg de carbonate de potassium dans 1 l de diméthylformamide (DMF) sont ajoutés successivement une solution de (±)-6-hydroxy-2,5,7,8-tétraméthylchromane-5-carboxylate de méthyle (préparation : cf. Scott et al., *Journal of the American Oil Chemist's Society*, May 1974, 200-203) et 1,2 kg de chlorure de benzyle. La suspension obtenue est agitée à 50 °C pendant 23 h. Au mélange ramené à 25 °C sont ajoutés rapidement 5 l d'eau et 4 l de *tert*-butylméthyléther. La phase organique est séparée et la phase aqueuse extraite à nouveau avec du *tert*-butylméthyléther (TBME). Les deux phases organiques sont combinées, lavées avec de l'eau, séchées sur

sulfate de sodium et le solvant éliminé par évaporation pour donner 1,1 kg d'une huile brune.
RMN $^1H$ (250 MHz, $CDCl_3$): 1,61 (s, $CH_3$); 1,80-2,00 et 2,35-2,70 (m, 2 $CH_2$); 2,12 (s, $CH_3$) ; 2,18 (s, $CH_3$) ; 2,22 (s, $CH_3$) ; 3,68 (s, $OCH_3$) ; 4,68 (s, $OCH_2$) ; 7,26-7,55 (m, 5 H aromatiques).

1.2) *acide (±)-6-benzyloxy-2,5,7,8-tétraméthylchromane-2-carboxylique* :

A une solution de 0,9 kg de (±)-6-benzyloxy-2,5,7,8-tétraméthylchromane-5-carboxylate de méthyle (obtenu à l'étape 1.1) dans 5 l de méthanol est ajoutée une solution aqueuse d'hydroxyde de sodium à 30%. Le mélange est agité à 25 °C pendant 1 h 30 puis 7 1 d'eau sont ajoutés rapidement. L'éthanol est éliminé par évaporation sous pression réduite (20 torrs) à une température maintenue entre 20 et 30 °C. 4 l de TBME sont ajoutés et le pH est ajusté à 1 par addition graduelle d'acide chlorhydrique concentré. La phase organique est séparée et la phase aqueuse extraite deux fois avec du TBME. Les trois phases organiques sont combinées, lavées avec de l'eau puis extraites avec une solution aqueuse d'hydroxyde de sodium à 10%. Cette phase aqueuse est alors à nouveau acidifiée et extraite avec du TBME. La phase organique récupérée est lavée avec de l'eau, séchée sur sulfate de sodium et le solvant éliminé par évaporation pour donner 0,9 kg d'une huile brune. Le produit est obtenu sous la forme d'un solide blanc analytiquement pur (point de fusion : 145-147 °C) après cristallisation dans un mélange TBME/heptane.
RMN $^1H$ (250 MHz, $CDCl_3$): 1,63 (s, $CH_3$); 1,84-1,90 et 2,35-2,77 (m, 2 $CH_2$); 2,13 (s, $CH_3$); 2,16 (s, $CH_3$); 2,22 (s, $CH_3$); 4,68 (s, $OCH_2$); 7,26-7,55 (m, 5 H aromatiques).

1.3) *acide (-)-6-benzyloxy-2,5,7,8-tétraméthylchromane-2-carboxylique* :

A une solution de 1 kg d'acide (±)-6-benzyloxy-2,5,7,8-tétraméthylchromane-2-carboxylique dans 5 l d'isopropanol (IPA) est ajoutée à une température de 65 °C une solution de 0,35 kg de (L)-(-)phényléthylamine. Une solution claire est obtenue. Le mélange est progressivement refroidi à 10 °C en 3 h (à vitesse de refroidissement constante) et agité pendant 2 h à cette température pour optimiser la cristallisation. Le solide est éliminé par filtration et recristallisé dans 7 l d'éthanol pour donner l'énantiomère S avec un excès énantiomérique supérieur à 99% (calculé par intégration / HPLC chirale). Le mélange obtenu est agité dans de l'acide chlorhydrique et extrait avec du TBME. La phase organique est séchée sur sulfate de sodium et le solvant éliminé sous pression réduite pour donner 250 g d'une huile qui cristallise rapidement (point de fusion : 161-162 °C ; excès énantiomérique mesuré par HPLC chirale : ee > 99%).
RMN $^1H$ (250 MHz, $CDCl_3$): 1,62 (s, $CH_3$); 1,84-1,89 et 2,35-2,75 (m, 2 $CH_2$) ; 2,12 (s, $CH_3$); 2,16 (s, $CH_3$); 2,22 (s, $CH_3$); 4,68 (s, $OCH_2$); 7,26-7,55 (m, 5 H aromatiques).
$[\alpha]_{20}^{D}$ (2% dans l'éthanol) : - 29,87.

**Exemple 2 : acide (+)-6-benzyloxy-2,5,7,8-tétraméthylchromane-2-carboxylique**

La solution mère obtenue lors de là cristallisation décrite dans l'étape 1.3 est réduite par évaporation du solvant sous pression réduite et recristallisée dans 3 l d'éthanol pour donner, après une procédure finale similaire celle décrite dans l'étape 1.3, 300 g d'une huile qui cristallise rapidement (point de fusion : 161-162 °C ; excès énantiomérique mesuré par RMN ou HPLC chirale : ee > 99%).
RMN $^1H$ (250 MHz, $CDCl_3$): 1,64 (s, $CH_3$); 1,84-1,90 et 2,35-2,77 (m, 2 $CH_2$); 2,14 (s, $CH_3$); 2,15 (s, $CH_3$); 2,22 (s, $CH_3$); 4,68 (s, $OCH_2$); 7,26-7,55 (m, 5 H aromatiques).
$[\alpha]_{20}^{D}$ (2% dans l'éthanol): + 29,75.

**Exemple 3 :**

3.1.) *(S)-3,4-dihydro-6-benzyloxy-2,5,7,8-tétraméthyl-2-{4-[(4-nitrophényl)-1-pipérazinyl]-carbonyl}-2H-1-benzopyrane :*

Une solution d'acide (-)-6-benzyloxy-2,5,7,8-tétraméthylchromane-2-carboxylique (obtenu à l'étape 1.3 ; 1 kg ; 2,937 mol) dans du 2-méthyltétrahydrofuranne (8,4 l) est agitée à température ambiante. Du 1,1'-earbonyldiimidazole (486 g; 2,996 mol) est ajouté et l'agitation maintenue pendant 2 h à température ambiante. De la 1-(4-nitrophénylpipérazine) (608,8 g ; 2,938 mol) est ajoutée et le mélange réactionnel est chauffé à 40 °C jusqu'à obtention d'une solution. Le mélange réactionnel est laissé revenir à température ambiante et l'agitation maintenue pendant une nuit. Le mélange réactionnel est refroidi à 0 °C et agité une heure durant à cette température puis filtré. Après séchage, un solide jaune est obtenu (1,344 kg ; rendement de 86%).
RMN $^1H$ (250 MHz, $CDCl_3$): 1,64 (s, 3H); 1,72-1,86 (m, 1H); 2,15 (s, 3H); 2,17 (s, 3H); 2,21 (s, 3H); 2,50-2,86 (m, 3H); 3,00-3,50 (m, 4H); 3,50-3,90 (m, 2H); 4,00-4,40 (m, 2H); 4,69 (s, 2H); 6,79 (d, 2H); 7,31-7,50 (m, 5 H) ; 8,13 (d, 2H).

3.2) *(S)-3,4-dihydro-2,5,7,8-tétraméthyl-2-{4-[(4-aminophényl)-1-pipérazinyl]-carbonyl}-2H-1-benzopyran-6-ol :*

Une solution du produit de l'étape 3.2 (1 kg ; 1,89 mol) dans du méthanol (10 l) est agitée à température ambiante avec Pd/C 10% (humide à 50%; 1,607 kg; 0,755 mol). Du cyclohexène (3,102 kg; 18,8 mol) est ajouté à cette suspension et le mélange réactionnel maintenu à reflux pendant 2 h. Le mélange résultant est filtré à travers un lit de clarcel et concentré à sec. Le composé attendu est obtenu (757,8 g ; rendement de 98%) et est immédiatement engagé dans l'étape suivante sans purification supplémentaire.
RMN $^1H$ (250 MHz, $CDCl_3$): 1,60 (s, 3H) ; 1,65-1,82 (m, 1H); 2,07 (s, 3H) ; 2,15 (s, 6H); 2,48-2,90 (m, 3H); 2,80-3,10 (d, 4H); 3,55-3,90 (m, 2H) ; 3,95-4,30 (m, 2H) ; 6,63 (d, 2H) ; 6,76 (d, 2H).

3.3) *dichlorhydrate de (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide*

Une solution de chlorhydrate de S-benzyl-(2-thiénylthioacétamidate) (701,5 g ; 2,6 mol) et du produit de l'étape 3.2 (409,5 g; 1 mol) dans de l'éthanol (4 l) est agitée à température ambiante pendant 2 h. Le mélange réactionnel est refroidi à 0 °C et agité une heure durant à cette température. Après filtration, de l'acide chlorhydrique 6N (2 mol) est ajouté au filtrat. Le mélange est concentré à sec et le résidu purifié dans du diméthylacétamide et de l'acétone. Le produit attendu est obtenu (355 g ; rendement de 60%).
RMN $^1H$ (250 MHz, DMSO): 1,54 (s, 3H); 1,58-1,75 (m, 1H); 1,99 (s, 3H); 2,06 (s, 3H); 2,10 (s, 3H); 2,30-2,74 (m, 3H); 2,90-3,45 (m, 4H); 3,45-3,90 (m, 2H) ; 3,50-3,90 (m, 2H) ; 3,90-4,40 (m, 2H) ; 7,10-7,40 (m, 5 H) ; 8,10-8,30 (dd, 2H) ; 8,75 (s, 1H) ; 9,80 (s, 1H) ; 11,57 (s, 1H).

## Revendications

**1.** A titre de produit industriel nouveau, un produit **caractérisé en ce qu'**il s'agit d'un produit de formule générale **(II)B**

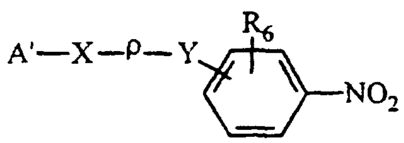

**(II)B**

sous forme racémique, d'énantiomères ou toute combinaison de ces formes, formule générale **(II)B** dans laquelle :

A' représente le radical

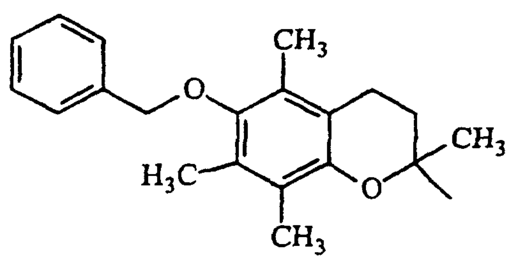

X représente $-Z_1$-CO- ;

ρ représente une liaison ou un hétérocycle choisi parmi le groupe constitué des radicaux pipéridine, pipérazine, homopipérazine, 2-méthylpipérazine, 2,5-diméthyl-pipérazine et 4-aminopipéridine ;

Y représente un radical choisi parmi les radicaux $-Z_2$- et $-NR_3-Z_2$,

$R_3$ représentant un atome d'hydrogène, un radical alkyle linéaire ou ramifié ayant de 1 à 6 atomes de carbone ou un radical $-COR_4$,

$R_4$ représentant un radical alkyle linéaire ou ramifié ayant de 1 à 6 atomes de carbone ;

$Z_1$ et $Z_2$ représentent indépendamment une simple liaison ou un radical alkylène linéaire ou ramifié ayant de 1 à 6 atomes de carbone, $Z_1$ et $Z_2$ représentant de préférence $-(CH_2)_m-$, m étant un entier compris entre 0 et 6 ;

et $R_6$ représente un atome d'hydrogène ou un groupe OH.

**2.** Produit selon la revendication 1, **caractérisé en ce qu'**il s'agit du (S)-3,4-dihydro-6-benzyloxy-2,5,7,8-tétraméthyl-2-{4-[(4-nitrophényl)-1-pipérazinyl]-carbonyl}-2H-1-benzopyrane.

**3.** Application du produit de formule générale **(II)B** à la préparation d'un produit de formule générale **(I)**

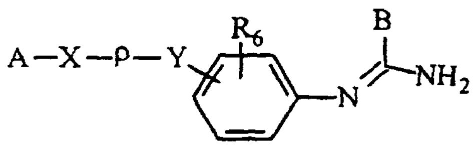

**(I)**

dans laquelle

A représente le radical

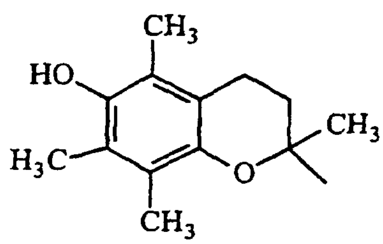

B représente un radical aryle carbocyclique ou aryle hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;

X représente $-Z_1-CO-$ ;

ρ représente une liaison ou un hétérocycle choisi parmi le groupe constitué des radicaux pipéridine, pipérazine, homopipérazine, 2-méthylpipérazine, 2,5-diméthyl-pipérazine et 4-aminopipéridine ;

Y représente un radical choisi parmi les radicaux $-Z_2-$ et $-NR_3-Z_2$,

$R_3$ représentant un atome d'hydrogène, un radical alkyle linéaire ou ramifié ayant de 1 à 6 atomes de carbone ou un radical $-COR_4$,

$R_4$ représentant un radical alkyle linéaire ou ramifié ayant de 1 à 6 atomes de carbone ;

$Z_1$ et $Z_2$ représentent indépendamment une simple liaison ou un radical alkylène linéaire ou ramifié ayant de

1 à 6 atomes de carbone, $Z_1$ et $Z_2$ représentant de préférence $-(CH_2)_m-$, m étant un entier compris entre 0 et 6 ;

$R_6$ représente un atome d'hydrogène ou un groupe OH ;

application **caractérisée en ce qu'**elle consiste à faire subir au produit de formule générale **(II)B** les étapes successives suivantes :

1) réduction catalytique du composé nitro de formule générale **(II)B**

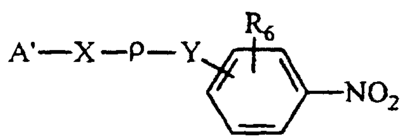

**(II)B**

dans un alcool inférieur, de préférence tel que méthanol, éthanol ou alcool isopropylique, par action d'un donneur d'hydrogène en présence de Pd/C pour donner le composé amino de formule générale **(III)**

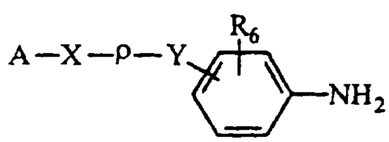

**(III)**

dans laquelle A, X, ρ, Y et $R_6$ ont la même signification que dans la formule générale **(I)** ;

2) réaction, dans un alcool inférieur, tel que méthanol, éthanol, alcool isopropylique ou t-butanol, de préférence dans l'alcool isopropylique, à une température comprise entre 20 et 90 °C, et durant une à 48 heures, éventuellement en présence de DMF, du composé de formule générale **(III)** décrit précédemment

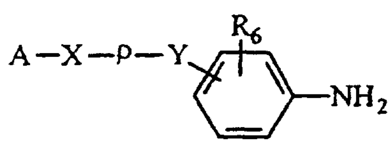

**(III)**

avec le composé de formule générale **(IV)**

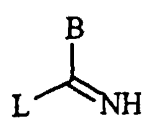

**(IV)**

dans laquelle B a la même signification que dans la formule générale **(I)** et L représente un groupe partant et notamment un radical alkoxy, alkylthio, aralkylthio, acide sulfonique, halogénure, alcool arylique ou tosyle, le composé de formule générale **(IV)** pouvant éventuellement être salifié par un acide minéral G.

4. Application selon la revendication 3, **caractérisée en ce que** le composé de formule générale **(IV)** employé dans l'étape 2) est le S-benzyl-(2-thiénylthioacétamidate).

5. Application selon la revendication 3, **caractérisée en ce que** le S-benzyl-(2-thiénylthioacétamidate) est utilisé sous une forme salifiée, de préférence sous sa forme chlorhydrate.

6. Application selon l'une des revendications 3 à 5, **caractérisée en ce que** le donneur d'hydrogène de l'étage 1) est le cyclohexène.

7. Application selon l'une des revendications 3 à 6, **caractérisée en ce que** le produit de formule générale **(I)** est le (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide.

## Claims

1. As a new industrial product, a product **characterized in that** it is a product of general formula **(II)B**

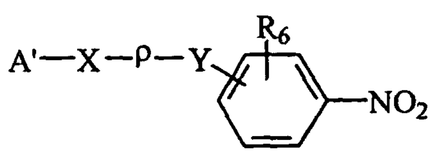

**(II)B**

in racemic, enantiomeric form or any combination of these forms,
general formula **(II)B** in which:

A' represents the

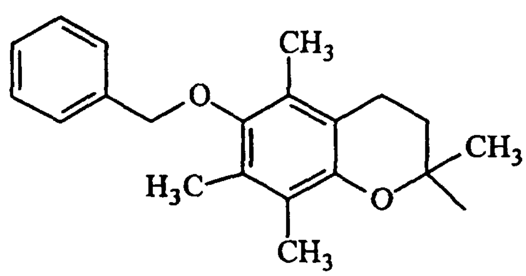

radical,

X represents -$Z_1$-CO-;

ρ represents a bond or a heterocycle chosen from the group constituted by the piperidine, piperazine, homopiperazine, 2-methylpiperazine, 2,5-dimethyl-piperazine and 4-aminopiperidine radicals;

Y represents a radical chosen from the -$Z_2$- and -$NR_3$-$Z_2$ radicals,

$R_3$ representing a hydrogen atom, a linear or branched alkyl radical having from 1 to 6 carbon atoms or a $-COR_4$ radical,

$R_4$ representing a linear or branched alkyl radical having from 1 to 6 carbon atoms;

$Z_1$ and $Z_2$ represent independently a single bond or a linear or branched alkylene radical having from 1 to 6 carbon atoms, $Z_1$ and $Z_2$ preferably representing $-(CH_2)_m-$, m being an integer between 0 and 6;

and $R_6$ represents a hydrogen atom or an OH group.

2. Product according to claim 1, **characterized in that** it is (S)-3,4-dihydro-6-benzyloxy-2,5,7,8-tetramethyl-2-{4-[(4-nitrophenyl)-1-piperazinyl]-carbonyl}-2H-1-benzopyrane.

3. Use of the product of general formula **(II)B** for the preparation of a product of general formula **(I)**

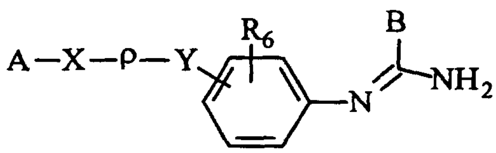

**(I)**

in which

A represents the

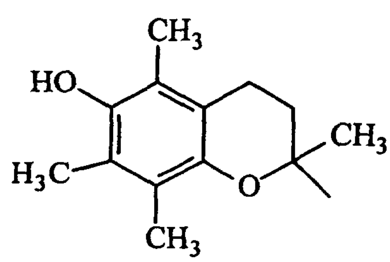

radical,

B represents a carbocyclic aryl or heterocyclic aryl radical with 5 or 6 members containing from 1 to 4 heteroatoms chosen from O, S, N and in particular the thiophene, furane, pyrrole or thiazole radicals, the aryl radical being optionally substituted by one or more groups chosen from the linear or branched alkyl, alkenyl or alkoxy radicals having 1 to 6 carbon atoms;

X represents $-Z_1-CO-$;

ρ represents a bond or a heterocycle chosen from the group constituted by the piperidine, piperazine, homopiperazine, 2-methylpiperazine, 2,5-dimethyl-piperazine and 4-aminopiperidine radicals;

Y represents a radical chosen from the $-Z_2-$ and $-NR_3-Z_2$ radicals,

$R_3$ representing a hydrogen atom, a linear or branched alkyl radical having from 1 to 6 carbon atoms or a $-COR_4$ radical,

$R_4$ representing a linear or branched alkyl radical having from 1 to 6 carbon atoms;

$Z_1$ and $Z_2$ independently represent a single bond or a linear or branched alkylene radical having from 1 to 6 carbon atoms, $Z_1$ and $Z_2$ preferably representing $-(CH_2)_m-$, m being an integer between 0 and 6;

$R_6$ represents a hydrogen atom or an OH group;

use **characterized in that** it consists of subjecting the product of general formula **(II)B** to the following successive stages:

1) catalytic reduction of the nitro compound of general formula **(II)B**

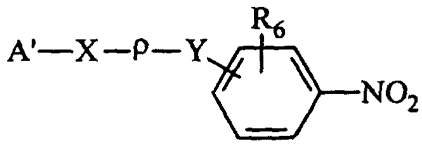

**(II)B**

in a lower alcohol, preferably such as methanol, ethanol or isopropyl alcohol, by the action of a hydrogen donor in the presence of Pd/C in order to produce the amino compound of general formula **(III)**

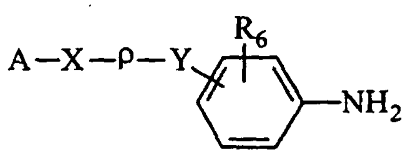

**(III)**

in which A, X, ρ, Y and $R_6$ have the same meaning as in general formula **(I)**;

2) reaction, in a lower alcohol, such as methanol, ethanol, isopropyl alcohol or t-butanol, preferably in isopropyl alcohol, at a temperature comprised between 20 and 90 °C, and lasting one to 48 hours, optionally in the presence of DMF, of the compound of general formula **(III)** described previously

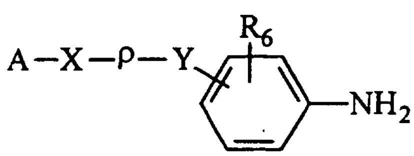

**(III)**

with the compound of general formula **(IV)**

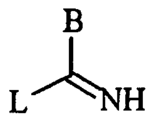

**(IV)**

in which B has the same meaning as in general formula **(I)** and L represents a leaving group and in particular an alkoxy, alkylthio, aralkylthio, sulphonic acid, halide, aryl alcohol or tosyl radical, the compound of general formula **(IV)** being optionally salified by a mineral acid G.

4. Use according to claim 3, **characterized in that** the compound of general formula **(IV)** used in Stage 2) is S-benzyl-(2-thienylthioacetamidate).

5. Use according to claim 3, **characterized in that** the S-benzyl-(2-thienylthioacetamidate) is used in a salified form, preferably in its hydrochloride form.

6. Use according to one of claims 3 to 5, **characterized in that** the hydrogen donor of Stage 1) is cyclohexene.

7. Use according to one of claims 3 to 6, **characterized in that** the product of general formula **(I)** is (S)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide.

**Patentansprüche**

1. Produkt in Form eines neuen Industrieprodukts, **dadurch gekennzeichnet, daß** es sich um ein Produkt der allgemeinen Formel (II)B

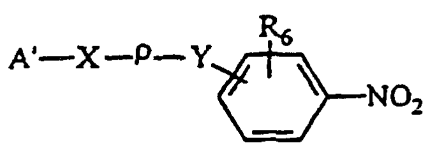

(II)B

in racemischer Form, in Form von Enantiomeren oder von jeder Kombination dieser Formen handelt, wobei in dieser allgemeinen Formel (II)B

A' den Rest

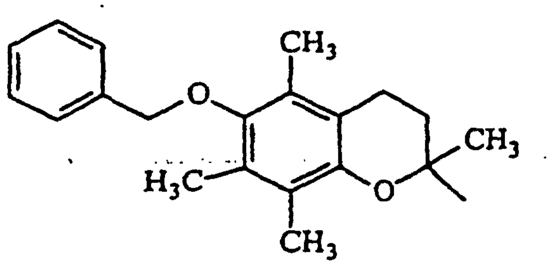

darstellt;

X $-Z_1$-CO- darstellt;

ρ eine Bindung oder einen Heterocyclus darstellt, der aus der Gruppe ausgewählt ist, die aus den Resten Piperidin, Piperazin, Homopiperazin, 2-Methylpiperazin, 2,5-Dimethylpiperazin und 4-Aminopiperidin besteht;

Y einen Rest darstellt, der aus den Resten $-Z_2$- und $-NR_3-Z_2$ ausgewählt ist,

wobei $R_3$ ein Wasserstoffatom, einen linearen oder verzweigten Alkylrest mit 1 bis 6 Kohlenstoffatomen oder einen Rest $-COR_4$ darstellt,

$R_4$ einen linearen oder verzweigten Alkylrest mit 1 bis 6 Kohlenstoffatomen darstellt;

$Z_1$ und $Z_2$ unabhängig voneinander eine Einfachbindung oder einen linearen oder verzweigten Alkylenrest mit 1 bis 6 Kohlenstoffatomen darstellen, wobei $Z_1$ und $Z_2$ vorzugsweise $-(CH_2)_m-$ darstellen, wobei m eine ganze Zahl zwischen 0 und 6 ist;

und $R_6$ ein Wasserstoffatom oder eine OH-Gruppe darstellt.

**2.** Produkt nach Anspruch 1, **dadurch gekennzeichnet, daß** es sich um (S)-3,4-Dihydro-6-benzyloxy-2,5,7,8-tetramethyl-2-{4-[(4-nitrophenyl)-1-piperazinyl]-carbonyl}-2H-1-benzopyran handelt.

**3.** Verwendung des Produkts der allgemeinen Formel (II)B für die Herstellung eines Produkts der allgemeinen Formel (I)

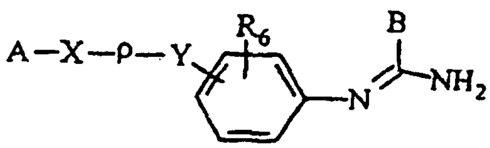

(I)

in der

A den Rest

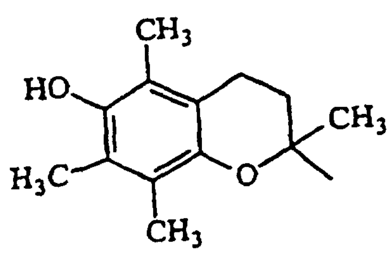

darstellt;

B einen carbocyclischen Arylrest oder einen heterocyclischen Arylrest mit 5 oder 6 Kettengliedern, die 1 bis 4 Heteroatome enthalten, die aus O, S, N ausgewählt sind, und insbesondere die Reste Thiophen, Furan, Pyrrol oder Thiazol darstellt, wobei der Arylrest gegebenenfalls durch eine oder mehrere Gruppen substituiert ist, die aus den linearen oder verzweigten Resten Alkyl, Alkenyl oder Alkoxy mit 1 bis 6 Kohlenstoffatomen ausgewählt sind;

X $-Z_1-CO-$ darstellt;

ρ eine Bindung oder einen Heterocyclus darstellt, der aus der Gruppe ausgewählt ist, die aus den Resten Piperidin, Piperazin, Homopiperazin, 2-Methylpiperazin, 2,5-Dimethylpiperazin und 4-Aminopiperidin besteht;

Y einen Rest darstellt, der aus den Resten $-Z_2-$ und $-NR_3-Z_2$ ausgewählt ist,

wobei $R_3$ ein Wasserstoffatom, einen linearen oder verzweigten Alkylrest mit 1 bis 6 Kohlenstoffatomen oder einen Rest $-COR_4$ darstellt;

$R_4$ einen linearen oder verzweigten Alkylrest mit 1 bis 6 Kohlenstoffatomen darstellt;

$Z_1$ und $Z_2$ unabhängig voneinander eine Einfachbindung oder einen linearen oder verzweigten Alkylenrest mit 1 bis 6 Kohlenstoffatomen darstellen, wobei $Z_1$ und $Z_2$ vorzugsweise $-(CH_2)_m-$ darstellen, wobei m eine ganze Zahl zwischen 0 und 6 ist;

$R_6$ ein Wasserstoffatom oder eine OH-Gruppe darstellt;

wobei diese Verwendung **dadurch gekennzeichnet ist, daß** sie darin besteht, daß man das Produkt der allgemeinen Formel (II)B den folgenden aufeinanderfolgenden Schritten unterzieht:

1) katalytische Reduktion der Nitroverbindung der allgemeinen Formel (II)B

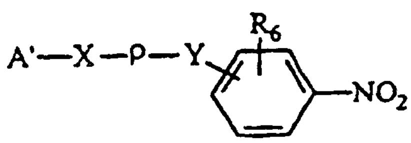

(II)B

in einem niederen Alkohol, vorzugsweise beispielsweise Methanol, Ethanol oder Isopropylalkohol, durch Einwirkung eines Wasserstoffdonators in Gegenwart von Pd/C, um die Aminoverbindung der allgemeinen Formel (III)

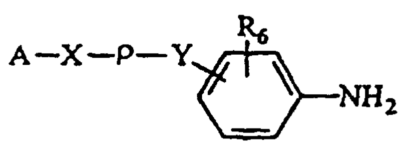

(III)

zu ergeben, in der A, X, ρ, Y und $R_6$ dieselbe Bedeutung wie in der allgemeinen Formel (I) haben;
2) Reaktion der Verbindung der oben beschriebenen allgemeinen Formel (III)

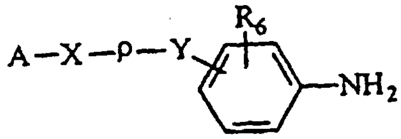

(III)

mit der Verbindung der allgemeinen Formel (IV)

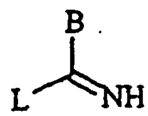

(IV)

in der B dieselbe Bedeutung hat wie in der allgemeinen Formel (I) und L eine Abgangsgruppe darstellt, und zwar insbesondere einen der Reste Alkoxy, Alkylthio, Aralkylthio, Sulfonsäure, ein Halogenid, Aryl- oder Tosylalkohol, wobei die Verbindung der allgemeinen Formel (IV) gegebenenfalls durch eine Mineralsäure G versalzt sein kann,

wobei die Reaktion in einem niederen Alkohol, wie z.B. Methanol, Ethanol, Isopropylalkohol oder t-Butanol, vorzugsweise in Isopropylalkohol, während einer bis 48 Stunden bei einer Temperatur zwischen 20 und 90°C ggf. in Gegenwart von DMF stattfindet.

**4.** Verwendung nach Anspruch 3, **dadurch gekennzeichnet, daß** die im Schritt 2) verwendete Verbindung der allgemeinen Formel (IV) S-Benzyl-(2-thienylthioacetamidat) ist.

**5.** Verwendung nach Anspruch 3, **dadurch gekennzeichnet, daß** das S-Benzyl-(2-thienylthioacetamidat) in Form eines Salzes, vorzugsweise in Form seines Chlorhydrats, verwendet wird.

**6.** Verwendung nach einem der Ansprüche 3 bis 5, **dadurch gekennzeichnet, daß** der Wasserstoffdonator des Schritts 1) Cyclohexen ist.

**7.** Verwendung nach einem der Ansprüche 3 bis 6, **dadurch gekennzeichnet, daß** das Produkt der allgemeinen Formel (I) (S)-N-{4-[4-[(3,4-Dihydro-6-hydroxy-2,5,7,8-tetramethyl-2H-1-benzopyran-2-yl)-carbonyl]-1-piperazinyl]phenyl}-2-thiophencarboximidamid ist.